(19) Europäisches Patentamt
European Patent Office
Office européen des brevets

(11) Veröffentlichungsnummer: **0 003 979**
**B1**

(12) **EUROPÄISCHE PATENTSCHRIFT**

(45) Veröffentlichungstag der Patentschrift: **21.10.81**

(51) Int. Cl.³: **A 61 F 1/00,** A 61 K 9/20

(21) Anmeldenummer: **79100452.6**

(22) Anmeldetag: **15.02.79**

(54) **Implantierbares Pharmaka-Depot und Verfahren zu dessen Herstellung.**

(30) Priorität: **20.02.78 DE 2807132**

(43) Veröffentlichungstag der Anmeldung:
**19.09.79 Patentblatt 79/19**

(45) Bekanntmachung des Hinweises auf die Patenterteilung:
**21.10.81 Patentblatt 81/42**

(84) Benannte Vertragsstaaten:
**BE CH FR GB IT LU NL SE**

(56) Entgegenhaltungen:
**DE - A - 2 163 034**
**DE - A - 2 620 890**
**DE - A - 2 620 891**

(73) Patentinhaber: **Battelle-Institut e.V.**
**Am Römerhof 35**
**D-6000 Frankfurt/Main 90 (DE)**

(72) Erfinder: **Reiner, Roland, Dr.**
**Gehspitz 11**
**D-6236 Eschborn (DE)**
Erfinder: **Kissing, Wolfgang, Dr.**
**Schillerstrasse 14**
**D-3440 Eschwege (DE)**
Erfinder: **Döring, Helga**
**Reischplatz 18**
**D-5000 Köln 21 (DE)**
Erfinder: **Köster-Lösche, Karl, Dr.**
**Westerfelder Weg 23**
**D-6390 Usingen (DE)**
Erfinder: **Heide, Helmut, Dr.**
**Am Hohenstein 14**
**D-6233 Kelkheim (DE)**

(74) Vertreter: **Blum, Klaus-Dieter, Dipl.-Ing.**
**Am Römerhof 35**
**D-6000 Frankfurt/Main 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 979 B1

# Implantierbares Pharmaka-Depot und Verfahren zu dessen Herstellung

Die Erfindung betrifft ein implantierbares Pharmaka-Depot zur Behandlung von Knochen- bzw. Knochenmarkerkrankungen, das aus einer Matrix, in der ein Wirkstoff eingelagert ist, besteht, sowie ein Verfahren zur Herstellung desselben. Das Pharmaka-Depot eignet sich insbesondere zur Prophylaxe und Therapie von Infektionskrankheiten des Knochens und des Knochenmarks, z.B. Osteomyelitis.

Zur Behandlung von infektiösen Knochenerkrankungen werden zur Zeit Depots auf der Basis von Methacrylsäureester/Acrylsäureester-Copolymerisaten eingesetzt, welche Wirkstoffe wie Sulfonamide, Antibiotika u.a. enthalten. Diese Polymerisate sind jedoch biologisch nicht abbaubar und müssen daher nach Beendigung der Therapie durch einen chirurgischen Eingriff entfernt werden.

Implantierbare Pharmaka-Depots bestehend aus einer keramischen Matrix und einem Wirkstoff sind bereits in der DE—A—2 163 034 beschrieben. Sie sind jedoch als Langzeit-Depots nicht geeignet.

Aus DE—A—2 620 890 und DE—A—2 620 891 sind ferner implantierbare Knochenersatzwerkstoffe auf der Basis von gesinterten Calciumphosphaten und bioresistenten oder resorbierbaren organischen Verbindungen bekannt. Die Calciumphosphate eignen sich jedoch allein als Matrixmaterialien für Langzeit-Depots nicht, da aufgrund des Resorptionsverhaltens die Wirkstoffabgabe äußerst rasch erfolgt.

Der Erfindung liegt daher die Aufgabe zugrunde, den Nachteil bekannter Depots zu überwinden und ein Pharmaka-Depot zu entwickeln, mit dem eine wirksame und verbesserte Therapeutika-Abgabe über einen längeren Zeitraum ermöglicht wird und bei dem eine nachträgliche Entfernung der Matrix nicht notwendig ist.

Es hat sich nun gezeigt, daß sich diese Aufgabe in technisch fortschrittlicher Weise lösen läßt, wenn gemäß vorliegender Erfindung das Matrixmaterial aus resorbierbaren gesinterten Calciumphosphaten besteht, die aus CaO und $P_2O_5$ in einem Mengenverhältnis von 5:1 bis 2:1, vorzugsweise 3:1, zusammengesetzt sind, und wenn das Depot zur Steuerung der Pharmaka-Abgabe-Kinetik Derivate der gesättigten oder ungesättigten aliphatischen Carbonsäuren mit 10 bis 20, vorzugsweise 15 bis 18 C—Atomen und/oder resorbierbare Polymerisate als Hilfsstoffe enthält. Weitere Einzelheiten sind in den Patentansprüchen 2 bis 5 beschrieben. Die Patentansprüche 6 und 7 betreffen ein bevorzugtes Verfahren zur Herstellung des erfindungsgemäßen Pharmaka-Depots.

Die erfindungsgemäß verwendeten resorbierbaren Calciumphosphate haben nicht nur die Fähigkeit, im Körper vollständig abgebaut zu werden, sie besitzen darüberhinaus Eigenschaften, die das Knochenwachstum positiv beeinflussen. Die Calciumphosphat-Matrix wird durch Reaktionssintern aus Calciumhydrogenphosphat und Calciumcarbonat hergestellt. Durch Variationen der Synthesebedingungen kann hierbei entweder die Tieftemperaturmodifikation ($\beta$-Whitlockit) oder die Hochtemperaturmodifikation ($\alpha$-Whitlockit) sowie Mischungen der beiden Modifikationen mit einer calciumphosphatischen Glasphase erhalten werden. Die Mengenanteile dieser Modifikationen in dem besamtgefüge beeinflussen die Resorptionsgeschwindigkeit des Werkstoffes. Sie können jedoch in Abhängigkeit von der voraussichtlichen Dauer der Wirkstoffversorgung in weiten Grenzen varriiert werden.

Die calciumphosphatische Matrix wird entweder in poröser fester Form oder in Pulverform mit den anderen Komponenten des erfindungsgemäßen Pharmaka-Depots kombiniert. Das Calciumphosphatpulver besitzt vorsugsweise eine Korngröße von $<200$ $\mu$m, während der Calciumphosphatkörper einen Porenanteil von 10 bis 25% aufweist. Eine Steuerung der Wirkstoffabgaberate ist durch geeignete Variation der Porenstruktur der Matrix ebenfalls möglich.

Das erfindungsgemäße Pharmaka-Depot enthällt einen Hilfsstoff zur Steuerung der Pharmaka-Abgabe-Kinetik und gegebenenfalls zur Stabilisierung des Matrixmaterials. Die Stabilisierung des Matrixmaterials ist insbesondere in den Fällen angezeigt, wenn man als Matrix Calciumphosphatpulver verwendet.

Als Hilfsstoffe werden Derivate der gesättigten oder ungesättigten aliphatischen Carbonsäuren mit 10 bis 20, vorzugsweise mit 15 bis 18 C—Atomen in einer Menge von bis 25, vorzugsweise 5 bis 20 Gew.-%, bezogen auf das Gesamtgewicht des Pharmaka-Depots, eingesetzt. Als solche sind z.B. Hexadecansäure, Octodecansäure, 9—Octodecensäure und Ölsäure geeignet. Bei den Derivaten der Carbonsäuren handelt es sich im wesentlichen um Alkali-, Erdalkali- und Aluminiumsalze sowie Triglyceride, Lipide oder Mischungen derselben. Als Alkali- bzw. Erdalkalisalze werden bevorzugt Natrium- und Calciumsalze verwendet.

Als Lipide kommen neutrale und nicht neutrale Phosphorlipide, vorzugsweise Lecithine, bzw. Glycolipide infrage. Auch Mischungen der genannten Derivate sind geeignet. Mischungen von Triglyceriden mit einem Schmelzpunkt von 20 bis 150°C mit Phosphorlipiden oder Mischungen der Calcium-, Aluminium- oder Natriumsalze der genannten Carbonsäuren mit Lecithinen werden bevorzugt eingesetzt.

Der in die Matrix einzulagernde Wirkstoff kann ferner mit einem filmbildenden, resorbierbaren Polymerisat in an sich bekannter Weise enkapsuliert sein. Es ist aber auch möglich, der Mischung sämtlicher Bestandteile des Depots das Monomere zuzusetzen, so daß in diesem Fall, nach erfolgter Polymerisation, das Matrixmaterial ebenfalls beschichtet wird. Geeignete Polymerisate sind z.B. Polycyanacrylate, Polyglycolsäurederivate, Polymilchsäurederivate. Die Beschichtung des Wirkstoffs bzw. des Gemisches aus dem Matrixmaterial und dem Wirkstoff trägt ebenfalls zur Steuerung der Wirkstoffabgaberate bei. Dadurch werden, z.B. bei hoher Serumlöslichkeit des Wirkstoffes, die Abgaberaten weitgehend verringert.

Zur Herstellung des erfindungsgemäßen Pharmaka-Depots kann man von einem Calciumphosphat-Pulver mit einer Korngröße von <200 $\mu$m ausgehen und dieses Pulver mit dem Wirkstoff, unter Zugabe genannter Hilfsstoffe, zu einer pastösen Masse vermischen. Die pastöse Masse kann erforderlichenfalls unter Anwendung von Wärme zu Tabletten verpreßt werden.

Wird jedoch von einem porösen, festen Calciumphosphatkörper ausgegangen, so sollte dieser zweckmäßigerweise einen Porenanteil von 10 bis 25 Gew-% aufweisen. Die porösen Calciumphosphatkörper werden zunächst mit einer Lösung des Wirkstoffes in einem ersten Lösungsmittel A getränkt. Das Lösungsmittel A wird zur Erzielung einer möglichst gleichmäßigen Ausfällung des Wirkstoffes innerhalb des porösen Körpers nicht durch Verdampfung entfernt, sondern durch ein zweites Lösungsmittel B, in dem der Wirkstoff nicht löslich ist, das jedoch mit dem ersten Lösungsmittel A zumindest begrenzt mischbar ist, ersetzt, welches dann verdampft werden kann. Gegebenenfalls kann dieser Tränkungsvorgang mehrfach wiederholt werden. Die Einbrungung der Hilfsstoffe in den Calciumphosphatkörper erfolgt ebenfalls durch Tränkung des Körpers mit einer Lösung der Hilfsstoffe in einem dritten Lösungsmittel C, das den Wirkstoff ebenfalls nicht löst, das aber mit dem zweiten Lösungsmittel B zumindest begrenzt mischbar ist. Beispielsweise bei einem wasserlöslichen Wirkstoff können als Lösungsmittel B u.a. Dioxan, Tetrahydrofuran, n-Butanol, Aceton und als Lösungsmittel C für die Hilfsstoffe z.B. Hexan verwendet werden.

Weitere Merkmale und Vorteile der Erfindung gehen aus der nachstehenden Schilderung von Ausführungsbeispielen hervor.

## Beispiel 1

Herstellung der Matrix:

a) Calciumhydrogenphosphat und Calciumcarbonat in feindisperser Form werden homogen vermischt und zu Tabletten verpreßt. Diese Tabletten werden anschließend 3 Stunden bei 1200°C gesintert. Nach der Gleichung

$$2\ CaHPO_4 + CaCO_3 \xrightarrow{1200°C} 3\ CaO \cdot P_2O_5 + H_2O + CO_2$$

entsteht Tricalciumphosphat in der Tieftemperaturmodifikation ($\beta$-Whitlockit). Die Tabletten werden zerkleinert und zu einem Pulver mit einer Korngröße von <65 $\mu$m vermahlen. Dieses Pulver kann bereits als Matrixmaterial verwendet werden. Durch Pressen des trockenen Pulvers, durch Schlickerguß oder Plastifizierung mit Wasser lassen sich beliebige Formen, z.B. Tabletten, herstellen.

b) Aus dem wie o.a. hergestellten Pulver wird durch Zusatz von 30 Gew.-% Wasser eine pastöse Masse gebildet, die in eine Lochform gestrichen wird. Nach dem Antrocknen werden die Calciumphosphatkörper, in Form von Zylindergranulaten mit einem Durchmesser von 5 mm und einer Höhe von 5 mm, aus der Form gestoßen und bei einer Temperatur von ca. 1500°C gesintert. Zur Erzeugung einer keramischen Bindung muß die Sintertemperatur so hoch gewählt werden, daß sich die Tieftemperaturmodifikation ($\beta$-Whitlockit) in die Hochtemperaturmodifikation des Tricalciumphosphats ($\alpha$-Whitlockit) umwandelt und sich außerdem eine Teilschmelze ausbildet, die die kristallinen Anteile miteinander verkittet. Durch eine gesteuerte, rasche Abkühlung des Sinterofens auf Zimmertemperatur werden die Hochtemperaturformen ($\alpha$-Whitlockit und Glasphase) zum Teil "eingefroren", so daß sich nur ein Teil dieser Phasen in die Tieftemperaturform zurück unwandelt. Auf diese Weise wird die Matrix hinsichtlich ihrer Zusammensetzung und Mikroporösität und demzufolge hinsichtlich ihrer biologischen Eigenschaften, wie Stabilität, Resorbierbarkeit, optimiert.

Die so hergestellten Matrix-Körper bzw. Granulate bestehen somit aus $\alpha$- und $\beta$-Tricalciumphosphat und der Glasphase. Der Werkstoff besitzt eine Dichte von 2,47 g/cm$^3$ und eine Raumerfüllung von ca. 78.6%.

c) Die wie o.a. hergestellten Granulate werden zu einem Pulver vermahlen und durch Absieben auf eine Korngröße von 65 $\mu$m klassiert.

Die folgenden Beispiele 2 bis 12 betreffen die Herstellung des erfindungsgemäßen Pharmaka-Depots.

## Beispiel 2

10 einzelne Calciumphosphatkörper, die nach dem Verfahren des Beispiels 1 b) hergestellt wurden, werden mit einer Lösung von Gentamycinsulfat in Wasser getränkt und sodann die Mischung durch 30-minütiges Evakuieren entgast. Anschließend wird zur Ausfällung des Gentamycinsulfats in dem porösen Körper Dioxan zugesetzt. Die Calciumphosphatkörper werden sodann in eine Lösung von Lecithin in Hexan gegeben und analog zu der Tränkung mit dem Wirkstoff durch Evakuieren entgast.

Mit einer Matrix, die 3,2 mg Gentamycinsulfat enthält, wird die Elutionsrate bestimmt. Hierfür werden die Pharmaka-Depots mit 5 ml eines 0,066 molaren Phosphatpuffers ($K_2HPO_4$/NaOH; pH 7,4) versetzt und bei 37°C stehengelassen. Der Puffer wird nach bestimmten Zeitabständen abgegossen und die Menge des abgegebenen Gentamycins gegen den Bacillus Subtilis gemessen. Nach 24 Stunden sind 21% des eingebrachten Gentamycins eluiert.

Beispiel 3

10 einzelne Calciumphosphatkörper, die nach dem Verfahren des Beispiels 1 b) hergestellt wurden, werden mit einer Lösung von Quinacrine-Dihydrochlorid in Wasser getränkt und der Wirkstoff durch Dioxan, THF, n-Butanol, Essigester, Aceton und Diglyme in den Körpern ausgefällt. Diese Körper werden sodann in eine Lösung von Lecithin in Hexan gegeben und analog zu der Tränkung mit dem Wirkstoff durch Evakuieren entgast.

Nach 48 Stunden beträgt die abgegebene Wirkstoffmenge 51% der im Calciumphosphatkörper befindiichen Menge. Bei einem vergleichbaren Pharmaka-Depot, das jedoch ohne Zusatz von Hilfsstoff hergestellt wird, beträgt die Wirkstoffabgabe im gleichen Zeitraum 92%.

Beispiel 4

8 g Calciumphosphatpulver, hergestellt nach dem Verfahren des Beispiels 1 c), werden mit 120 mg Chloramphenicol und 200 g gehärtetem Erdnußöl bei 50°C zu einer Paste verrieben und zu 10 Tabletten verpreßt. Die Wirkstoffabgabe beträgt 25% nach 48 Stunden.

Beispiel 5 bis 11

Tabletten mit einem Gewicht von ca. 1 g werden durch Vermischen von Calciumphosphatpulver, 12 mg Chloramphenicol bzw. Gentamycin und Hilfsstoffen durch Verpressen zu Tabletten mit einem Durchmesser von 13 mm und einer Höhe von 3,5 mm unter einem Druck von 130 kp/cm² hergestellt. Die Wirkstoffabgaberaten sind in der nachfolgenden Tabelle zusammengefaßt.

Beispiel 12

13 mg Chloramphenicol und 850 mg Calciumphosphatpulver werden vermischt und anschließend 120 mg Methylcyanacrylat zugesetzt. Tabletten mit einem Gewicht von ca. 1 g werden nach dem Verfahren der Beispiele 5 bis 11 hergestellt. Die Wirkstoffabgaberate beträgt 8,5% nach 24 Stunden und 10% nach 42 Stunden.

Wenn aber Chloramphenicol und Methylcyanacrylat zuerst vermischt und erst nach der Anpolymerisation des Monomeren Calciumphosphatpulver zugesetzt wird, beträgt die Wirkstoffabgaberate nach 24 Stunden 18% und nach 42 Stunden 23%.

| Beispiel | Wirkstoff | Hilfsstoff Gew.-% bezogen auf Gesamtgewicht | Gew.-% akkumulierte Wirkstoffabgabe in Stunden | | | | | | | | |
|---|---|---|---|---|---|---|---|---|---|---|---|
| | | | 12 | 24 | 48 | 70 | 96 | 120 | 148 | 172 | 288 |
| 5 | Chloramphenicol | – | 60 | 76 | 92 | 94 | 95 | 98 | – | – | – |
| 6 | ,, | 10 Ca-Stearat | – | 13 | 19 | – | – | 31 | 35 | 38 | 50 |
| 7 | ,, | 20 Al-Stearat | – | 9 | 17 | – | – | 32 | 38 | 43 | 60 |
| 8 | ,, | 10 Al-Distearat | – | 17,5 | 28 | – | – | 45 | 52 | 56 | 71 |
| 9 | Gentamycin | 20 Ca-Stearat | – | 9 | 14 | 18 | 22 | 24 | 27 | 29 | 35 |
| 10 | ,, | 10 Ca-Stearat | – | 21 | 29 | 34 | 36 | 38 | 39 | 40 | 41 |
| 11 | ,, | 20 Al-Palmitat | – | 17 | 25 | 28 | 30 | 32 | 33 | 34 | 35 |

## Patentansprüche

1. Implantierbares Pharmaka-Depot zur Behandlung von Knochen- bzw. Knochenmarkerkrankungen, bestehend aus einer keramischen Matrix, in der ein Wirkstoff eingelagert ist, dadurch gekennzeichnet, daß das Matrixmaterial aus resorbierbaren gesinterten Calciumphosphaten besteht, die aus CaO und $P_2O_5$ in einem Mengenverhältnis von 5:1 bis 2:1, vorzugsweise 3:1, zusammengesetzt sind, und daß das Depot zur Steuerung der Pharmaka-Abgabe-Kinetik Derivate der gesättigten oder ungesättigten aliphatischen Carbonsäuren mit 10 bis 20, vorzugsweise 15 bis 18 C—Atomen und/oder resorbierbare Polymerisate als Hilfsstoffe enthält.

2. Pharmaka-Depot nach Anspruch 1, dadurch gekennzeichnet, daß die Derivate der aliphatischen Carbonsäuren in einer Menge von bis 25 Gew.-%, vorzugsweise 5 bis 20 Gew.-% bezogen auf das Gesamtgewicht des Pharmaka-Depots, verwendet werden.

3. Pharmaka-Depot nach Anspruch 1 oder 2, dadurch gekennzeichnet, daß als Derivate der Carbonsäuren Alkali-, Erdalkali- oder Aluminiumsalze, Triglyceride, Lipide, vorzugsweise Phosphorlipide, Lecithine bzw. Glycolipide oder Mischungen derselben verwendet werden.

4. Pharmaka-Depot nach Anspruch 3, dadurch gekennzeichnet, daß Triglyceride mit einem Schmelzpunkt von 20 bis 150°C, vorzugsweise in Mischung mit Phosphorlipiden verwendet werden.

5. Pharmaka-Depot nach Anspruch 3, dadurch gekennzeichnet, daß Calcium-, Aluminium- oder Natriumsalze gesättigter sowie ungesättigter aliphatischer Carbonsäuren mit 15 bis 18 C—Atomen, vorzugsweise in Mischung mit Lecithinen verwendet werden.

6. Verfahren zur Herstellung des Pharmaka-Depots nach einem der Ansprüche 1 bis 5, dadurch gekennzeichnet, daß man einen porösen festen Calciumphosphatkörper mit einem Porenanteil von 10 bis 25% in an sich bekannter Weise herstellt, ihn mit einer Lösung des Wirkstoffes in einem ersten Lösungsmittel A tränkt und den Wirkstoff innerhalb des porösen Gefüges durch ein zweites Lösungsmittel B ausfällt, wobei der Wirkstoff in dem Lösungsmittel B unlöslich ist, aber das Lösungsmittel B mit dem Lösungsmittel A zumindest begrenzt mischbar ist, und daß man nach der Tränkung des Calciumphosphatkörpers mit dem Wirkstoff eine weitere Tränkung desselben mit einer Lösung von Hilfsstoffen in einem dritten Lösungsmittel C vornimmt, wobei der Wirkstoff in dem dritten Lösungsmittel C ebenfalls nicht löslich, aber mit dem Lösungsmittel B zumindest begrenzt mischbar ist und anschließend das Lösungsmittel C durch Verdunstung entfernt.

7. Verfahren nach Anspruch 6, dadurch gekennzeichnet, daß man den Wirkstoff vor oder während der Einbringung in die Matrix mit einem resorbierbaren und filmbildenden Polymerisat in an sich bekannter Weise enkapsuliert.

## Revendications

1. Support pharmaceutique implantable destiné au traitement des maladies des os ou de la moelle des os, constitué d'un matrice céramique dans laquelle est incorporée une matière active, caractérisé en ce que la matrice est constituée par une matière composée de phosphates de calcium frittés résorbables, qui sont composés de CaO et de $P_2O_5$ dans le rapport en quantités de 5:1 à 2:1, et de préférence de 3:1, ce support contenant, pour diriger la cinétique de libération du produit pharmaceutique des dérivés des acides carboxyliques aliphatiques saturés ou insaturés à 10 à 20 atomes de carbone, de préférence 15 à 18 atomes de C, et/ou des polymèrisats résorbables comme produits auxiliaires.

2. Support phamaceutique suivant la revendication 1, caractérisé en ce que l'on utilise les dérives des acides carboxyliques dans une proportion pouvant aller jusqu'à 25% et de préférence de 5 à 20%, calculée sur le poids total de ce support.

3. Support pharmaceutique suivant l'une des revendications 1 et 2 caractérisé en ce que l'on utilise comme dérivés des acides carboxyliques des sels alcalins, alcalino-terreux ou d'aluminium, des triglycérides, des lipides, de préférence des phospholipides, de la lécithine ou des glucolipides, ou des mélanges de ces produits.

4. Support pharmaceutique suivant la revendication 3, caractérisé en ce que l'on utilise des triglycérides dont le point de fusion se situe entre 20 et 150°C, de préférence en mélange avec des phospholipide.

5. Support pharmaceutique suivant la revendication 3, caractérisé en ce que l'on utilise des sels de calcium, d'aluminium ou de sodium des acides carboxyliques aliphatiques saturés ou insaturés à 15 à 18 atomes de carbone, de préférence en mélange avec des lécithines.

6. Procédé pour la fabrication du support pharmaceutique suivant l'une quelconque des revendications 1 à 5, caractérisé en ce que l'on fabrique des pièces solides poreuses en phosphate de calcium, où la proportion de pores est de 10 à 25% d'une façon connue, l'imprègne d'une solution de la matière active dans un premier solvant A, et fait précipiter la matière active à l'intérieur de la structure poreuse au moyen d'un deuxième solvant B, la matière active étant insoluble dans ce solvent B, mais ce solvant B étant au moins dans certaines limites miscible avec le solvant A, et qu'après l'imprégnation de la pièce en phosphate de calcium avec le matière active, on procède à une autre imprégnation de cette pièce avec une solution de produits auxiliaires dans un troisième solvant C, la matière active étant

également insoluble dans ce troisième solvant C, mais miscible au moins dans certaines limites avec le solvant B, et enfin on élimine le solvant C par évaporation.

7. Procédé suivant la revendication 6, caractérisé en ce que l'on encapsule, d'une façon connue, la matière active avant ou pendant l'introduction dans la matrice, dans un polymérisat résorbable et formant un film.

**Claims**

1. An implantable drug depot for the treatment of diseases of the bone or bone marrow consisting of a ceramic matrix in which an active ingredient is incorporated characterized in that the matrix material consists of resorbable sintered calcium phosphates composed of CaO and $P_2O_5$ in a ratio between 5:1 and 2:1, preferably 3:1, and said depot contains derivatives of saturated and unsaturated aliphatic carboxylic acids having 10 to 20, preferably 15 to 18 carbon atoms and/or resorbable polymers as adjuvant agents to control the kinetics of the drug release.

2. Drug depot as claimed in claim 1 wherein the derivatives of aliphatic carboxylic acids are used in an amount up to 25 weight percent, preferably between 5 and 20 weight percent, based on the total weight of the drug depot.

3. Drug depot as claimed in claim 1 or 2 wherein alkaline, alkaline earth or aluminium salts, triglycerides, lipids, preferably phospholipids, lecithins or glycolipids, or the mixtures thereof are used as derivatives of the carboxylic acids.

4. Drug depot as claimed in claim 3 wherein triglycerides with a melting point between 20 and 150°C, preferably in mixture with phospholipids are used.

5. Drug depot as claimed in claim 3 wherein calcium, aluminium or sodium salts of saturated and unsaturated aliphatic carboxylic acids having 15 to 18 carbon atoms, preferably in mixture with lecithins, are used.

6. Process for the production of the drug depot according to one of the claims 1 to 5, which comprises the forming a porous solid body of calcium phosphate having a pore ratio of 10 to 25 percent in a known manner, impregnating said body with a solution of an active ingredient in a first solvent A, precipitating the active ingredient within the porous body using a second solvent B, the solvent B being mixable with the solvent A at least to a limited extent, and the active ingredient being insoluble in solvent B, impregnating the body of calcium phosphate subsequent to its impregnation with the active ingredient with a solution of the adjuvant agents in a third solvent C, the active ingredient being insoluble in the third solvent C being mixable with the solvent B at least to a limited extent and removing the solvent C by evaporation.

7. Process as claimed in claim 6 wherein the active ingredient is encapsulated in a known manner in a resorbable and film-forming polymer before or during the incorporation of the active ingredient into the matrix.